Europäisches Patentamt

European Patent Office

Office européen des brevets

(11) Numéro de publication: **0 402 187**
**A1**

## (12) DEMANDE DE BREVET EUROPEEN

(21) Numéro de dépôt: 90401275.4

(51) Int. Cl.⁵: **A61B 17/36**

(22) Date de dépôt: **15.05.90**

(30) Priorité: **19.05.89 FR 8906545**

(43) Date de publication de la demande:
**12.12.90 Bulletin 90/50**

(84) Etats contractants désignés:
**BE CH DE FR GB IT LI NL SE**

(71) Demandeur: **A L M**
**52, avenue Lénine**
**F-93230 Romainville(FR)**

(72) Inventeur: **Villain, Claude**
**22, allée de Champereau, Chevry**
**F-91190 Gif-Sur-Yvette(FR)**

(74) Mandataire: **Le Moenner, Gabriel**
**L'AIR LIQUIDE, Société Anonyme pour**
**l'etude et l'exploitation des procédés**
**Georges Claude 75, Quai d'Orsay**
**F-75321 Paris Cédex 07(FR)**

(54) **Bras optique amovible pour l'acheminement d'un faisceau laser.**

(57) Un bras optique pour l'acheminement d'un faisceau laser comprend une succession de tubes (3, 4, 5) articulés entre eux à l'aide de paliers anti-frictions (18, 19) et dans lesquels se propage le faisceau; plusieurs miroirs de renvoi axial (11, 12) disposés chacun à l'entrée de l'un des tubes; une pièce d'extrémité d'entrée (1) du faisceau laser montée de façon amovible sur la sortie d'un dispositif générateur de faisceau laser; et une pièce d'extrémité de sortie (2) du faisceau adaptée pour coopérer avec un dispositif manuel d'application du faisceau laser. Ce bras optique comporte des moyens de freinage en rotation (29, 47) coopérant avec les deux premiers tubes articulés (3, 4) au voisinage de l'extrémité d'entrée du faisceau et un moyen d'équilibrage (34) monté sur le second tube articulé (4) à compter de ladite extrémité d'entrée.

FIG.2

EP 0 402 187 A1

La présente invention concerne un bras optique amovible constitué par une succession de tubes articulés entre eux avec des miroirs de renvoi axial pour l'acheminement d'un faisceau laser utilisé notamment dans le domaine médical.

On connaît, par exemple, des appareils à laser $CO_2$ utilisés en thérapie chirurgicale, telle que la dermatologie, l'urologie, la gynécologie et la chirurgie plastique. Ces appareils à laser sont équipés de bras optiques assurant l'acheminement de deux faisceaux laser coaxiaux : un faisceau laser $CO_2$ de traitement avec une longueur d'onde égale à 10,6 $\mu$m et un faisceau laser rouge hélium-néon (HeNe) de longueur d'onde égale à 0,6 $\mu$m pour la visée.

Les bras optiques sont constitués d'une succession de tubes articulés entre eux dans lesquels se propagent les faisceaux laser. A l'entrée de chacun de ces tubes est disposé un miroir de renvoi axial à 45°.

Ces bras optiques sont en outre amovibles et peuvent être enlevés et remis en place sans qu'il soit nécessaire de procéder à un réglage de centrage des faisceaux. En période d'inutilisation de l'appareil à laser, le bras est replié et fixé le long de l'appareil.

Selon la technique antérieure, le bras optique est équilibré par un contre-poids agissant au niveau de la deuxième articulation des tubes à compter de l'extrémité d'entrée du faisceau laser. Ce contre-poids est généralement lourd et encombrant, et a pour effet de rendre le bras peu esthétique, difficile à démonter et à ranger. Lorsque le bras optique est lâché, il peut facilement bouger dans l'espace et son extrémité libre, sur laquelle est monté un dispositif d'application de faisceau laser, peut s'éloigner de la zone de travail du chirurgien.

L'objet de la présente invention est de pallier ces inconvénients par la réalisation d'un bras optique amovible équilibré, compact, léger à manipuler et facile à ranger.

Selon l'invention, on réalise un bras optique amovible pour l'acheminement d'un faisceau laser comprenant une succession de tubes articulés entre eux à l'aide de paliers anti-frictions et dans lesquels se propage le faisceau laser, et plusieurs miroirs de renvoi axial disposés chacun à l'entrée de l'un des tubes, une pièce d'extrémité d'entrée du faisceau laser étant montée de façon amovible sur la sortie d'un dispositif générateur de faisceau laser et une pièce d'extrémité de sortie du faisceau laser étant adaptée pour coopérer avec un dispositif manuel d'application du faisceau laser, tel qu'une pièce à main, un micromanipulateur ou une colposcope.

Le bras optique de l'invention comporte des moyens de freinage en rotation coopérant avec au moins deux des tubes, et des moyens d'équilibrage sur au moins un des tubes.

De préférence, au moins un des tubes est équipé à la fois d'un moyen d'équilibrage et d'un moyen de freinage en rotation. D'une manière avantageuse, les deux premiers tubes situés au voisinage de la pièce d'extrémité d'entrée du faisceau sont munis des moyens de freinage en rotation et le second tube articulé à partir de ladite pièce d'extrémité d'entrée muni d'un moyen d'équilibrage.

Selon un mode préféré de réalisation de l'invention, le moyen d'équilibrage du bras est constitué par un ressort hélicoïdal exerçant un effort de rappel en torsion sur le second tube articulé. Les moyens de freinage peuvent être constitués par des joints annulaires de friction.

De préférence, les paliers anti-frictions sont constitués par des paliers à roulement. La partie interne du second tube, montée solidairement sur des bagues intérieures de roulement correspondant, est mobile en rotation par rapport à une partie fixe solidaire des bagues extérieures du roulement. Le ressort peut être disposé autour de la partie fixe et présenter une extrémité immobile par rapport à la partie fixe. L'autre extrémité du ressort est alors solidaire de la partie interne du tube par l'intermédiaire d'une bague auxiliaire par exemple.

La bague auxiliaire peut être rendue solidaire de la partie interne du second tube à l'aide de vis de pression traversant radialement la bague auxiliaire et coopérant avec une rainure annulaire de la partie interne du second tube.

Le second tube peut comporter en outre une enveloppe tubulaire rendue solidaire de la bague auxiliaire au moyen de vis de fixation radiales par exemple, et munie d'une lumière circonférentielle à l'une de ses extrémités. La lumière circonférentielle coopère avec une butée escamotable radialement, de façon à délimiter l'angle de rotation de la partie interne dudit tube par rapport à la partie fixe.

Grâce au ressort hélicoïdal logé à l'intérieur de l'enveloppe du second tube articulé, le bras optique peut être maintenu en équilibre dans l'espace. La butée escamotable empêche un éventuel mouvement du bras vers une position indésirable pour le travail du chirurgien.

Le repliage et le rangement du bras le long de l'appareil de traitement thérapeutique peut s'effectuer après avoir pressé ladite butée escamotable ce qui autorise une rotation d'amplitude plus grande du second tube articulé.

La présence des joints annulaires de friction sur les deux premiers tubes articulés permet d'éviter des mouvements désordonnés du bras dans l'espace. Le bras peut en effet rester immobile à l'endroit où il est lâché, ce qui facilite considérablement le travail du chirurgien.

Il est à noter que les joints de friction sont avantageusement montés de sorte qu'ils n'altèrent pas la souplesse générale et l'articulation du bras.

On peut également alléger le bras optique en utilisant un matériau composite, tel que des fibres de carbone, pour réaliser en partie ou entièrement les tubes articulés, sans diminuer la rigidité de ces tubes. De tels tubes réalisés en fibres de carbone présentent en outre l'avantage d'être ininflammables et résistants aux liquides médicaux couramment utilisés.

D'autres caractéristiques de l'invention ressortiront de la description détaillée d'un exemple de réalisation de l'invention pris à titre nullement limitatif et illustré par les dessins annexés, sur lesquels :

la figure 1 est une vue schématique d'un bras optique selon l'invention; et

la figure 2 est une vue partielle en coupe axiale du bras optique de l'invention au voisinage de son extrémité d'entrée du faisceau laser.

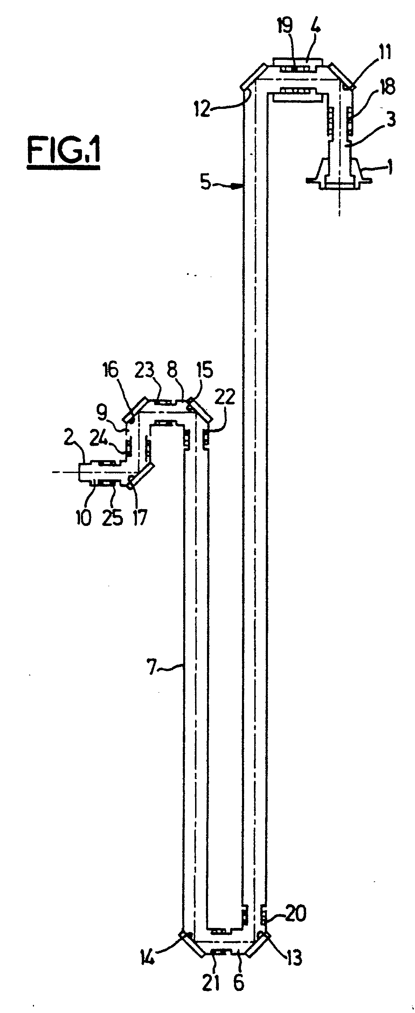

Tel qu'il est illustré sur la figure 1, le bras optique présente une pièce d'extrémité d'entrée 1 de faisceau laser qui peut être assemblée de façon amovible à un dispositif générateur de faisceau laser non représenté, et une pièce d'extrémité de sortie 2 pouvant également être montée de façon amovible à un dispositif d'application de faisceau laser non représenté. Le faisceau laser est acheminé par le bras optique depuis la pièce d'extrémité d'entrée 1 jusqu'à la pièce d'extrémité de sortie 2.

Le bras optique comporte une succession de tubes articulés référencés de 3 à 10 depuis la pièce d'extrémité d'entrée 1 du faisceau laser. Chaque tube articulé est perpendiculaire aux tubes voisins immédiats. A chaque angle formé par deux tubes articulés consécutifs est placé un miroir, référencé de 11 à 17, de renvoi axial du faisceau laser, orienté à 45° par rapport à l'axe respectif des tubes articulés.

Les tubes articulés 3 à 10 sont séparés des tubes voisins par des paliers anti-friction, référencés de 18 à 25, par exemple des paliers à roulement ou des paliers lisses. Ces paliers permettent une articulation facile des tubes du bras optique.

Comme on peut le voir sur la figure 1, le faisceau laser, schématisé par le trait mixte, entre dans le bras optique par la pièce d'extrémité d'entrée 1, traverse coaxialement et successivement les tubes 3 à 10 tout en étant réfléchi par les miroirs 11 à 17 situés aux angles formés par les tubes consécutifs, et sort enfin du bras optique par la pièce d'extrémité de sortie 2 qui est solidaire du dernier tube articulé 10.

Les troisième et cinquième tubes 5 et 7 à compter de la pièce d'extrémité d'entrée 1 présentent chacun une longueur nettement plus importante que les autres tubes 3, 4, 6 et 8 à 10. Le bras optique ainsi constitué peut présenter des configurations diverses pour répondre aux exigences du chirurgien pendant son travail.

On peut utiliser un matériau composite, par exemple les fibres, de carbone, pour la réalisation des tubes du bras optique, notamment les tubes de grande longueur 5 et 7, ce qui permet de réduire le poids du bras optique dans son ensemble tout en conservant une grande rigidité des tubes correspondants.

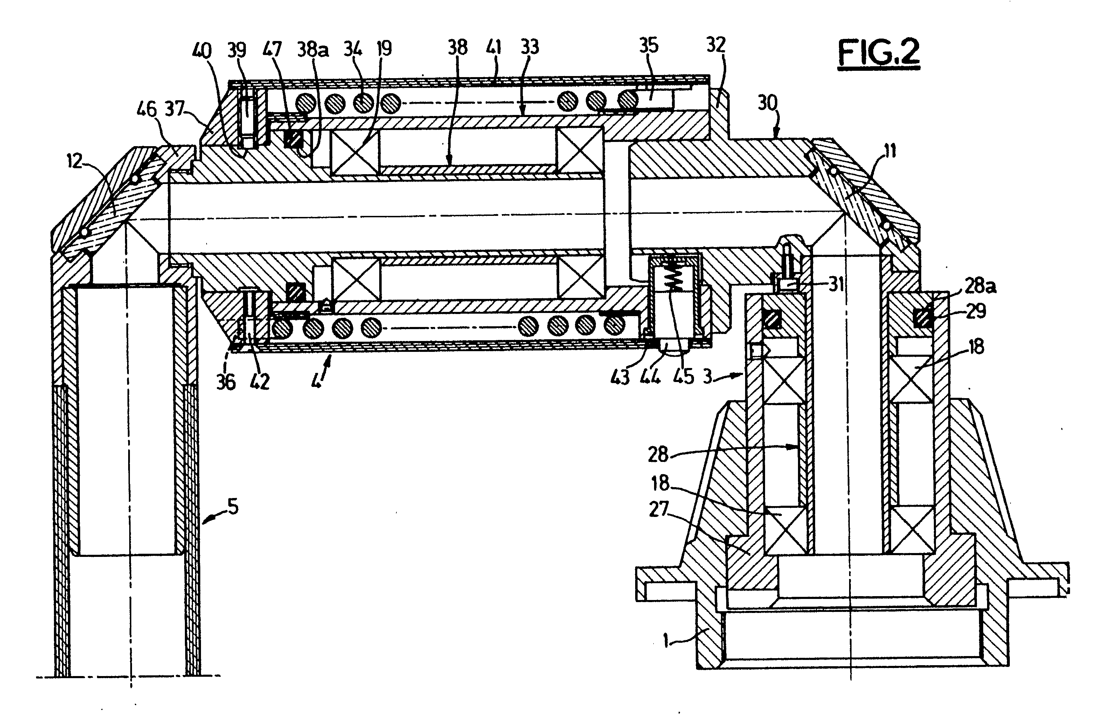

On va décrire maintenant, en se référant à la figure 2, la structure du bras optique, conforme de l'invention, au niveau des deux premiers tubes articulés 3 et 4.

La pièce d'extrémité d'entrée 1 du bras optique est disposée autour et rendue solidaire de la partie externe 27 du premier tube articulé 3, et est montée de façon amovible sur une embase d'un dispositif générateur de faisceau laser (non représenté). La partie interne 28 du tube 3 est mobile en rotation par rapport à la partie externe 27 grâce aux deux roulements 18 écartés l'un de l'autre.

Un joint torique de friction 29 est disposé dans une gorge annulaire 28a au voisinage de l'extrémité supérieure de la partie interne 28 et est en contact permanent avec la face interieure de la partie externe 27. Le joint de friction 29 crée ainsi un freinage en rotation entre les parties interne 28 et externe 27 du premier tube articulé 3.

Une pièce tubulaire 30 coudée à 90° présente une extrémité rendue solidaire de l'extrémité supérieure de la partie interne 28 du premier tube articulé 3 à l'aide de vis de fixation 31. Le miroir de renvoi axial 11 est encastré à 45° par rapport à l'axe du premier tube 3 et est situé à l'angle extérieur de la pièce coudée 30. La pièce coudée 30 présente, au voisinage de sa seconde extrémité, un collet 32 sur lequel est montée la partie externe 33 du second tube articulé 4. Le montage est réalisé à l'aide de vis parallèles au second tube 4, non représentées. Les tubes 3 et 4 sont alors perpendiculaires l'un par rapport à l'autre.

Autour de la partie externe 33 est disposé un ressort hélicoïdal 34 d'équilibrage dont une extrémité 35 est immobilisée par rapport à la partie externe 33, au moyen par exemple d'un logement pratiqué à l'extrémité de la partie 33 proche du collet 32. L'autre extrémité 36 du ressort 34 est immobilisée par rapport à une bague auxiliaire 37 située à l'opposé du collet 32, et rendue solidaire de la partie interne 38 du second tube articulé 4. Le montage de la bague auxiliaire 37 autour de la partie interne 38 peut être réalisé au moyen de vis de pression 39 traversant la bague auxiliaire 37 et coopérant avec une rainure annulaire 40 pratiquée sur la partie interne 38.

Autour du ressort 34 est disposée une enveloppe tubulaire 41 dont une extrémité est fixée sur la bague auxiliaire 37 au moyen de vis de fixation 42.

Sur l'autre extrémité de l'enveloppe 41 est pratiquée une lumière circonférentielle 43 dans laquelle se trouve logée une butée escamotable 44 dont le déplacement radial est rendu possible grâce à un ressort de compression 45.

La butée 44 permet de délimiter, en fonction de la longueur de la lumière 43, l'amplitude de rotation de l'enveloppe 41, de la bague auxiliaire 37 et enfin de la partie interne 38 par rapport à la partie externe 33 du second tube 4. Une pièce coudée 46 est solidaire d'une extrémité du troisième tube articulé 5 et fixée sur la partie interne 38 du second tube articulé 4. La lumière 43 de l'enveloppe 41 et la butée escamotable 44 déterminent en conséquence l'amplitude de déplacement angulaire du tube 5 par rapport au tube 4.

Comme pour le premier tube articulé 3, le second tube articulé 4 est pourvu d'un joint torique de friction 47 logé dans une gorge annulaire 38a de la partie interne 38 et est en contact de frottement avec la face intérieure de la partie externe 33, de façon à créer un freinage en rotation entre les parties interne 38 et externe 33 du second tube articulé 4.

Lorsque la butée escamotable 44 est enfoncée, l'enveloppe 41 peut passer au-dessus de la butée 44, dans le prolongement de la lumière 43, l'amplitude de rotation entre les parties interne 38 et externe 33 du tube 4 étant alors augmentée, ceci dans le but par exemple de replier le bras optique dans une position appropriée de rangement le long de l'appareil de traitement thérapeutique équipé du bras optique de l'invention.

Le faisceau laser, schématisé par le trait mixte, coïncide sensiblement avec les axes des tubes respectifs en suivant un trajet dévié à 90° chaque fois qu'il rencontre un miroir de renvoi axial. Le parcours du faisceau laser à l'intérieur du bras optique est déterminé par la position respective des tubes articulés.

Selon le mode de réalisation de l'invention illustré sur la figure 2, lorsque la pièce d'extrémité d'entrée 1 est montée sur l'appareil à laser, la partie externe 27 du premier tube 3 est immobilisée par rapport à l'appareil à l'aide de la pièce d'entrée 1. Grâce aux paliers à roulement 18, la partie interne 28 du premier tube 3 peut tourner sur elle-même en entraînant la rotation de la pièce coudée 30 autour de l'axe du premier tube 3, ce qui permet la rotation du second tube 4 et le reste du bras optique autour de l'axe du premier tube 3. Le joint de friction 29 crée un frottement entre les parties interne 28 et externe 27 du premier tube 3 pour amortir le mouvement de rotation.

Le second tube 4 présente la partie externe 33 fixe par rapport à la pièce coudée 30 et la partie interne 38 pouvant tourner sur elle-même en entraînant en rotation la pièce coudée 46 et le troisième tube 5 autour de l'axe du second tube 4. Le joint de friction 47 permet d'amortir cette rotation autour de l'axe du second tube 4.

Par ailleurs, la vis de pression 39, coopérant avec la rainure annulaire 40 de la partie interne 38 du second tube 4, rend la bague intermédiaire 37 solidaire de la partie interne 38. L'extrémité 36 du ressort 34, logée dans la bague intermédiaire 37, tourne autour de l'axe du second tube 4 avec la partie interne 38. L'autre extrémité 35, logée dans la partie externe 33, demeure fixe par rapport à celle-ci pendant que la partie interne 38 est en rotation autour de l'axe du second tube 4. Le mouvement de rotation de la partie interne 38 par rapport à la partie externe 33 provoque une déformation du ressort 34 qui réagit, en conséquence, en créant un effort de rappel en torsion sur les parties interne 38 et externe 33 du second tube 4. La combinaison des efforts du ressort 34 et du joint de friction 47 permet de régler le mouvement de rotation autour de l'axe du second tube 4 et d'obtenir un équilibrage approprié du bras optique dans son ensemble.

Le fait d'associer les joints de friction 29, 47 et le ressort de rappel 34 aux deux tubes consécutifs permet de contrôler le mouvement d'articulation du bras optique selon deux axes perpendiculaires.

La lumière circonférentielle 43 pratiquée sur l'enveloppe 41, en coopération avec la butée escamotable 44, permet de définir l'écart angulaire maximal autorisé pour la rotation de la partie interne 38 autour de l'axe du second tube 4 pendant le travail du chirurgien.

## Revendications

1. Bras optique amovible pour l'acheminement d'un faisceau laser, comprenant une succession de tubes (3 à 10) articulés entre eux à l'aide de paliers anti-frictions (18 à 25) et dans lesquels se propage le faisceau; plusieurs miroirs de renvoi axial (11 à 17) disposés chacun à l'entrée de l'un des tubes; une pièce d'extrémité d'entrée (1) du faisceau laser montée de façon amovible sur la sortie d'un dispositif générateur de faisceau laser; et une pièce d'extrémité de sortie (2) du faisceau adaptée pour coopérer avec un dispositif manuel d'application du faisceau laser, caractérisé par le fait qu'il comporte des moyens de freinage en rotation (29, 47) coopérant avec au moins deux tubes articulés (3, 4), et des moyens d'équilibrage (34) montés sur au moins un des tubes articulés (4).

2. Bras optique selon la revendication 1, caractérisé par le fait qu'au moins un des tubes (4) est équipé des moyens de freinage en rotation (47) et des moyens d'équilibrage (34).

3. Bras optique selon la revendication 1 ou 2, caractérisé par le fait que les deux premiers tubes (3, 4) au voisinage de la pièce d'extrémité d'entrée du faisceau sont équipés des moyens de freinage en rotation (29, 47).

4. Bras optique selon l'une quelconque des revendications précédentes, caractérisé par le fait que les moyens d'équilibrage sont constitués par un ressort hélicoïdal exerçant un effort de rappel en torsion.

5. Bras optique selon l'une quelconque des revendications précédentes, caractérisé par le fait que les moyens de freinage sont constitués par des joints annulaires de friction.

6. Bras optique selon l'une quelconque des revendications précédentes, caractérisé par le fait que les paliers anti-frictions sont constitués par des paliers à roulement (18 à 25).

7. Bras optique selon l'une quelconque des revendication 3 à 6, caractérisé par le fait que la partie interne (38) du second tube (4), solidaire des bagues intérieures de roulement correspondant (19), est mobile en rotation par rapport à une partie fixe (33) solidaire des bagues extérieures du roulement (19), et que le ressort hélicoïdal est disposé autour de la partie fixe et présente une extrémité (35) immobile par rapport à la partie fixe, l'autre extrémité (36) du ressort étant solidaire de la partie interne par l'intermédiaire d'une bague auxiliaire (37).

8. Bras optique selon la revendication 7, caractérisé par le fait que la bague auxiliaire (37) est solidaire de la partie interne du second tube à l'aide de vis de pression (39) traversant radialement la bague auxiliaire et coopérant avec une rainure annulaire (40) de la partie interne du tube.

9. Bras optique selon la revendication 7 ou 8, caractérisé par le fait que le second tube (4) comporte en outre une enveloppe tubulaire (41) solidaire de la bague auxiliaire et munie d'une lumière circonférentielle (43) à une extrémité, ladite lumière coopérant avec une butée escamotable (44), de façon à délimiter l'angle de rotation de la partie interne (38) autour de l'axe dudit tube.

10. Bras optique selon l'une quelconque des revendications précédentes, caractérisé par le fait qu'il comporte des tubes articulés en matériau composite.

# FIG.1

FIG.2

EP 0 402 187 A1

# RAPPORT DE RECHERCHE EUROPEENNE

Office européen des brevets

Numero de la demande

EP 90 40 1275

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-3 913 582 (U. SHARON) <br> * abrégé; colonne 1, ligne 48 - colonne 2, ligne 50; colonne 3, ligne 34-54; colonne 3, ligne 64 - colonne 4, ligne 31; colonne 7, ligne 13-15; figures 1,4,5; revendications 1,4 * <br> --- | 1 | A 61 B 17/36 |
| Y | DE-U-8 535 100 (FIRMA CARL ZEISS) <br> * revendications 1,3,6; page 2, ligne 8 - page 3, ligne 4; page 3, paragraphe 3; figures 1a-c * <br> --- | 1 | |
| A | FR-A-2 518 764 (ESSILOR INTERNATIONAL) <br> * page 5, lignes 24-38; page 7, ligne 34 - page 8, ligne 2; figures 1-4; revendications 1 * <br> --- | 1,6 | |
| A | US-A-4 623 229 (L. GALAN) <br> * abrégé; colonne 5, lignes 17-38; colonne 6, lignes 21-31; figures 3,5 * <br> --- | 1,5,6 | |
| A | FR-A-2 337 538 (MOCHIDA PHARMACEUTICAL) <br> * page 6, ligne 27 - page 7, ligne 21; revendications 1,2,6-8; figures 3,4 * <br> --- | 1,3,6,8 | DOMAINES TECHNIQUES RECHERCHES (Int. Cl.5) <br><br> A 61 B <br> B 23 K <br> F 16 L <br> G 02 B |
| A | US-A-4 667 081 (P.S. TURIN et al.) <br> * abrégé; figures 3,5; colonne 8, lignes 31-53 * <br> --- | 1,5 | |
| A | DE-U-8 619 188 (FIRMA CARL ZEISS) <br> * page 3, paragraphe 1; page 4, paragraphe 6; figure 1a * <br> ----- | 9 | |

Le présent rapport a été établi pour toutes les revendications

| Lieu de la recherche | Date d'achèvement de la recherche | Examinateur |
|---|---|---|
| BERLIN | 22-08-1990 | HYLLA W R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

EPO FORM 1503 03.82 (P0402)